# EUROPEAN PATENT APPLICATION

(11) **EP 1 008 575 A1**
(43) Date of publication of application: **14.06.2000**
(21) Application number: 99309810.2
(22) Date of filing: 07.12.1999
(51) Int. Cl.: C07C 17/20, C07C 19/12

(54) **Preparation of 1-chloro-2,2-difluoroethane ("142")**

(30) Priority: 08.12.1998 US 207473
(71) Applicant: ELF ATOCHEM NORTH AMERICA, INC., Philadelphia Pennsylvania 19102-3222 (US)
(72) Inventor: Chen, Bin, Wayne, Pennsylvania 19087 (US); Bolmer, Michael S., Collegeville, Pennsylvania 19426 (US); Elsheikh, Maher Y., Wayne, Pennsylvania 19087 (US)
(74) Representative: Stoner, Gerard Patrick

(57) **Abstract**

A process for the preparation of 1-chloro-2,2-difluoroethane ("142") by fluorinating 1,1,2-trichloroethane in the gas phase with HF in the presence of a fluorination catalyst. 142 is a known foam blowing agent.

## Description

### BACKGROUND

This invention relates to the preparation of 1-chloro-2,2-difluoroethane (142) from 1,1,2-trichloroethane ("140"), particularly to processes wherein said 140 is fluorinated with hydrogen fluoride ("HF") in the gas phase in the presence of a fluorination catalyst. 1-Chloro-2,2-difluoroethane is known to have utility as a foam blowing agent.

Applicant is not aware of literature disclosing reactions between 140 and HF to produce 142. Attempts to react 140 with HgF₂ have been reported; for example, A. L. Henne et al, in the Journal of the American Chemical Society, Vol. 58, pages 889-890 (1936), reported that reacting 140 with HgF₂ gave an 8-10% yield of 142. Since 142 is useful as a low ozone depleting blowing agent, it would be useful to have a process for readily preparing the same in good yield.

### BRIEF SUMMARY OF THE INVENTION

A process for preparing 142 is provided, which process comprises (a) contacting 140 with HF in the gas phase in the presence of a fluorination catalyst (preferably the fluorinated salt of chromium oxide, Cr₂O₃) under conditions sufficient to produce 142, and (b) recovering 142 from the resultant reactant mixture.

### DETAILED DESCRIPTION

It has now been discovered that 142 can be successfully prepared in good yields by the catalyzed, gas phase reaction of 140 and HF, wherein the catalyst is typically a supported or unsupported fluorinated salt of one or more of chromium, iron, niobium, nickel, antimony, tin, tantalum and titanium, preferably the fluorinated salt of chromium oxide, Cr₂O₃. Useful catalyst supports include fluorinated Al₂O₃, activated carbon, graphite or fluorinated graphite. The initial catalyst salts can be formed from chlorides, nitrates, and the like, while the initial catalyst support can be, for example, Al₂O₃, since the catalyst and support are fluorinated with HF either during an initial catalyst activation or during the course of the reaction. HF activation is well known in the art, typically involving a procedure wherein HF and air or nitrogen are fed over a heated catalyst bed for about 18 hours.

Typical conditions for carrying out the reaction are a temperature between 100°C and 380°C (preferably between 120°C and 250°C), a pressure of between 0 psig and 400 psig (preferably between 50 psig and 200 psig), a feed ratio of HF to 140 of between 2 and 20 (preferably between 5 and 10) and a contact time of between 1 and 100 seconds (preferably between 10 and 35 seconds).

The hydrogen chloride (HCl) by-product may be removed by distillation or absorption into water or caustic solution. The unreacted HF can be separated by distillation, absorption into water or caustic solution, phase separation or by use of a semipermeable membrane. Other organic by-products may be separated by distillation. Unsaturated organic by-products may be more easily separated by first reacting the product mixture with chlorine or bromine before distillation.

The practice of the invention is illustrated in more detail in the following nonlimiting example: Chromium oxide (Cr₂O₃) catalyst (20 cc, 27 g) was activated at 380°C. by cofeeding a mixture of HF (6.6 g/h) and air (100 cc/min) over the catalyst bed for 18 hours. 140 and HF, in a molar ratio of HF:140 of about 7.8:1 (10.1 g/h of HF and 0.1 cc/min of 140), were then fed to the reactor in the gas phase at 220°C. and 150 psig for a contact time of 34 seconds, resulting in 100% conversion of the 140, with selectivity being 70.2% for the desired 142. Analysis was done by a gas chromatograph, equipped with a thermal conductivity detector and a flame ionization detector.

## Claims

1. A process for preparing 1-chloro-2,2-difluoroethane which comprises (a) contacting 1,1,2-trichloroethane with hydrogen fluoride in the gas phase in the presence of a fluorination catalyst to produce 1-chloro-2,2-difluoroethane; and (b) recovering 1-chloro-2,2-difluoroethane from the resultant reaction mixture in step (a).

2. A process as in Claim 1 wherein the catalyst is the fluorinated salt of chromium oxide.
